# EUROPEAN PATENT SPECIFICATION

(11) **EP 2 917 168 B1**
(45) Date of publication and mention of the grant of the patent: **27.06.2018**
(21) Application number: 13852846.8
(22) Date of filing: 11.11.2013
(51) Int. Cl.: C07C 2/04, C07C 4/06, C07C 11/06, C10G 11/18, C10G 50/00, C10G 57/02, C10G 11/05

(54) **FLUID CATALYTIC CRACKING PROCESS**
FLUID-CATALYTIC-CRACKING-VERFAHREN
PROCÉDÉ DE CRAQUAGE CATALYTIQUE DE FLUIDE

(30) Priority: 12.11.2012 US 201261725231 P
(43) Date of publication of application: 16.09.2015
(73) Proprietor: UOP LLC, Des Plaines, Illinois 60017-5017 (US)
(72) Inventor: WEGERER, David A., Des Plaines, Illinois 60017-5017 (US); VANDEN BUSSCHE, Kurt M., Des Plaines, Illinois 60017-5017 (US); KRUSE, Todd M., Des Plaines, Illinois 60017-5017 (US); MEHLBERG, Robert L., Des Plaines, Illinois 60017-5017 (US); FEI, Zhihao, Des Plaines, Illinois 60017-5017 (US)
(74) Representative: Boult Wade Tennant
(86) International application number: PCT/US2013/069406
(87) International publication number: WO 2014/074975

(56) References cited:
- WO-A1-89/12036
- US-A- 5 389 232
- US-A- 5 895 830
- US-A1- 2001 040 118
- US-A1- 2005 121 361
- US-A1- 2010 158 767
- US-B1- 6 222 087

## Description

### FIELD OF THE INVENTION

This invention generally relates to a process for fluid catalytic cracking.

### DESCRIPTION OF THE RELATED ART

Increasing the propene production in a fluid catalytic cracking (hereinafter may be abbreviated as "FCC") unit can require the recycle of one or more C4⁺ alkenes back to one or more risers in the fluid catalytic cracking unit for further processing. One option for achieving a higher overall propene yield from an FCC unit is to oligomerize the available butenes in a C4 product stream and send the oligomerized product back to the main FCC riser to crack the product to propene. However, mixing this oligomerized product with the fresh feed may not optimize cracking of the oligomerized product. High residence times associated with the fresh feed in the riser can crack the oligomerized product without optimizing the selectivity of propene. Additionally, the oligomerized product may predominantly crack back to butenes. Hence, this blending of the oligomerized product with the feed may not optimize the differences in cracking rates to produce an optimal mix of products. Also, the production of high octane gasoline may suffer, as the desired branched C8 species are cracked. As a consequence, there is a desire to develop alternative schemes for removing this shortcoming.

US2005/0121361 discloses a fluid catalytic cracking process.

### SUMMARY OF THE INVENTION

The present invention is a process for fluid catalytic cracking according to claim 1.

Typically, feeding the oligomerized product separately from the feed at a higher location on the riser shortens the residence time. As such, a higher selectivity to both propene and/or a high octane gasoline product can be achieved. The lower residence time can provide a greater selectivity by cracking each feed into the desired species. Generally, these species, such as a linear oligomerized product, may crack with a higher selectivity to propene and have a higher cracking rate. What is more, the lower residence time can prevent the cracking of highly branched oligomerized product that may predominantly crack back to butenes. The high selectivity to propene from cracking more linear species can preserve the highly branched species that may desirably be blended into the gasoline pool due to their high octane value. The embodiments herein can also reduce the recycling of butenes by reducing the cracking of oligomerized product back to butenes, and instead producing from the oligomerized product a high selectivity for high octane gasoline species and propene.

### DEFINITIONS

As used herein, the term "stream" can be a stream including various hydrocarbon molecules, such as straight-chain, branched, or cyclic alkanes, alkenes, alkadienes, and alkynes, and optionally other substances, such as gases, e.g., hydrogen, or impurities, such as heavy metals, and sulfur and nitrogen compounds. The stream can also include aromatic and non-aromatic hydrocarbons. Moreover, the hydrocarbon molecules may be abbreviated C1, C2, C3...Cn where "n" represents the number of carbon atoms in the one or more hydrocarbon molecules. Generally, a stream characterized by a hydrocarbon abbreviation, e.g., a C4 stream, may be rich in that numerated hydrocarbon, e.g., hydrocarbons having four carbon atoms, but other numerated hydrocarbons may also be present, e.g., hydrocarbons having 3 and/or 5 carbon atoms.

As used herein, the term "zone" can refer to an area including one or more equipment items and/or one or more sub-zones. Equipment items can include one or more reactors or reactor vessels, heaters, exchangers, pipes, pumps, compressors, and controllers. Additionally, an equipment item, such as a reactor, dryer, or vessel, can further include one or more zones or sub-zones.

As used herein, the term "rich" can mean an amount of generally at least 40%, and preferably 70%, by mole, of a compound or class of compounds in a stream.

As used herein, the term "substantially" can mean an amount of generally at least 80%, preferably 90%, and optimally 99%, by mole, of a compound or class of compounds in a stream.

As used herein, the term "riser-reactor" generally means a reactor used in a fluid catalytic cracking process that can include a riser and a separation vessel. Usually, such a riser-reactor operates by providing catalyst at the bottom of a riser that proceeds to a separation vessel having a mechanism for separating the catalyst from a hydrocarbon.

As used herein, the term "heavy naphtha" can mean a hydrocarbon material boiling in a range of 85 to 190° C, and can include one or more C6-C10 hydrocarbons.

As used herein, the term "light cycle oil" can hereinafter be abbreviated "LCO" and may refer to a hydrocarbon material boiling in a range of 204 to 343° C, and can include one or more C13-C18 hydrocarbons.

As used herein, the term "heavy cycle oil" can hereinafter be abbreviated "HCO" and may refer to a hydrocarbon material boiling in a range of 343 to 524° C, and can include one or more C16-C25 hydrocarbons.

As used herein, the term "FCC bottoms" can mean a hydrocarbon material boiling in a range of 370 to 575° C, and can include one or more C22-C45 hydrocarbons.

As used herein, the terms "volume of riser", "first feed injection point", "second feed injection point", and "volumetric flow rate" can be abbreviated as "VR", "FFIP", "SFIP", and "VFR". Generally, the feed injection point is the location that a feed is provided to a riser, often through a distributor.

As used herein, the term "residence time" can mean the amount of time a particle of a feed remains in a riser and, e.g., can be calculated for a first feed and a second feed as, respectively:
First Feed Residence Time = (VR above FFIP)/(VFR of First Feed + VFR of Second Feed)
Second Feed Residence Time = (VR above SFIP)/(VFR of First Feed + VFR of Second Feed)

As used herein, the terms "alkanes" and "paraffins" may be used interchangeably.

As used herein, the terms "alkenes" and "olefins" may be used interchangeably.

As used herein, the specified alkenes can include their isomers. As an example, the term "butene" can include 2-methylpropene, 1-butene, and 2-butene. Similarly, alkenes such as pentene and hexene can include their respective isomers as well.

As used herein, the term "kilopascal" may be abbreviated "kPa" and all pressures disclosed herein are absolute.

As used herein, the term "weight percent" may be abbreviated as "wt%".

As used herein, the process flow lines in the figures can be referred to interchangeably as, e.g., lines, feeds, mixtures, effluents, portions, parts, products, or streams.

### BRIEF DESCRIPTION OF THE DRAWINGS

FIG. 1 is a schematic depiction of an exemplary unit for processing hydrocarbons.
FIG. 2 is a schematic depiction of an exemplary FCC apparatus.
FIG. 3 is a graphical depiction of propene yield versus residence time.
FIG. 4 is a graphical depiction propene yield based on vacuum gas oil weight percent versus vacuum gas oil conversion.

### DETAILED DESCRIPTION

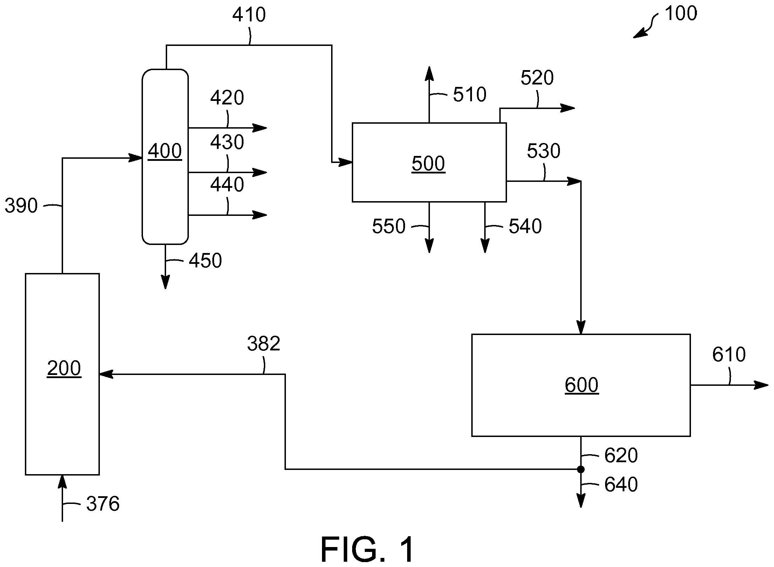

Referring to FIG. 1, an exemplary unit 100 for processing hydrocarbons can include an FCC apparatus 200, a separation zone 400, another separation zone 500, and an oligomerization reaction zone 600. Generally, a first feed 376, as hereinafter described, can be provided to the FCC apparatus 200, which can, in turn, provide an effluent 390.

The effluent 390 can be sent to a separation zone 400, which can include at least one of one or more distillation columns, one or more membranes, one or more flash drums, and one or more receivers. The separation zone 400 may provide a one or more C7⁻ hydrocarbon stream 410, a heavy naphtha stream 420, an LCO stream 430, an HCO stream 440, and an FCC bottoms stream 450. The one or more C7⁻ hydrocarbon stream 410 may be provided to the another separation zone 500, and can include one or more light alkenes. Particularly, the one or more light alkenes may include at least one C3-C6 alkene, preferably at least one C4-C5 alkene. The one or more C7⁻ hydrocarbon stream 410 may also include one or more alkanes, such as one or more C3-C6 alkanes.

The another separation zone 500 may include at least one of one or more distillation columns, one or more membranes, one or more flash drums, and one or more receivers. The another separation zone 500 can produce a fuel gas stream 510, one or more C3 hydrocarbons stream 520, one or more C4 hydrocarbons stream 530, one or more C5-C7 hydrocarbons stream 540, and one or more C7⁺ hydrocarbons stream 550. Although the zones 400 and 500 are depicted separately, it should be understood that these zones 400 and 500 may be combined into a single zone. Conversely, the zone 400 and/or 500 may be split into further zones or sub-zones. At least some of the equipment for such separation zones 400 and/or 500 are disclosed in, e.g., US 3,470,084. The one or more C4 hydrocarbons stream 530 can be provided to the oligomerization reaction zone 600. Although the one or more C4 hydrocarbons stream 530 can be rich in one or more C4 hydrocarbons, the stream 530 can include one or more C3-C6 hydrocarbons, preferably one or more C4 and C5 hydrocarbons, and optimally one or more C4 hydrocarbons. Often the stream 530 can include alkenes and alkanes, typically light alkenes, such as propene, butene, pentene, and hexene, preferably butene and pentene.

One exemplary oligomerization reaction zone 600 can be operated at a temperature of 30° to 300°C, preferably 160° to 220°C, and a pressure of 790 to 8,400 kPa, preferably 3,400 to 6,400 kPa. One preferred catalyst may be a solid phosphoric acid (hereinafter may be abbreviated "SPA") catalyst. The SPA catalyst refers to a solid catalyst that contains as a principal ingredient an acid of phosphorous such as ortho-, pyro- or tetraphosphoric acid.

An SPA catalyst can be formed by mixing the acid of phosphorous with a siliceous solid carrier to form a wet paste. This paste may be calcined and then crushed to yield catalyst particles where the paste may be extruded or pelleted prior to calcining to produce more uniform catalyst particles. Often, the carrier is a naturally occurring porous silica-containing material such as kieselguhr, kaolin, infusorial earth, and diatomaceous earth. A minor amount of various additives such as mineral talc, fuller's earth, and iron compounds including iron oxide may be added to the carrier to increase its strength and hardness. The combination of the carrier and the additives can include 15 to 30% of the catalyst, with the remainder being the phosphoric acid. The additive may include 3 to 20% of the total carrier material.

The oligomerization zone 600 contains a different oligomerization catalyst, which includes a zeolitic catalyst. The zeolite may comprise 5 to 95%, by weight, of the catalyst. Suitable zeolites may include zeolites having a structure from one of the following classes: MFI, MEL, SFV, SVR, ITH, IMF, TUN, FER, EUO, BEA, FAU, BPH, MEI, MSE, MWW, UZM-8, MOR, OFF, MTW, TON, MTT, AFO, ATO, and AEL. These three letter codes for structure types are assigned and maintained by the International Zeolite Association Structure Commission in the Atlas of Zeolite Framework Types, which is at http://www.iza-structure.org/databases/. In a preferred aspect, the oligomerization catalyst has a zeolite with a framework having a ten-ring pore structure. Examples of suitable zeolites having a ten-ring pore structure include those comprising TON, MTT, MFI, MEL, AFO, AEL, EUO and FER. In a further preferred aspect, the oligomerization catalyst including a zeolite having a ten-ring pore structure includes an uni-dimensional pore structure. Generally, an uni-dimensional pore structure indicates zeolites containing non-intersecting pores that are substantially parallel to one of the axes of the crystal. The pores preferably extend through the zeolite crystal. Suitable examples of zeolites having a ten-ring uni-dimensional pore structure may include MTT. The oligomerization catalyst includes an MTT zeolite. The MTT zeolite may be obtained from Zeolyst International located in Conshohocken, PA.

The first oligomerization catalyst may be formed by combining the zeolite with a binder, and then forming the catalyst into pellets. The pellets may optionally be treated with a phosphoric reagent to create a zeolite having a phosphorous component of 0.5 to 15%, by weight, of the treated catalyst. Typically, the binder is used to confer hardness and strength on the catalyst. Generally, binders include alumina, aluminum phosphate, silica, silica-alumina, zirconia, titania and combinations of these metal oxides, and other refractory oxides, and clays such as montmorillonite, kaolin, palygorskite, smectite and attapulgite. A preferred binder is an aluminum-based binder, such as alumina, aluminum phosphate, silica-alumina and clays.

One of the components of the catalyst binder may be alumina. The alumina source may be any of the various hydrous aluminum oxides or alumina gels such as alpha-alumina monohydrate of the boehmite or pseudo-boehmite structure, alpha-alumina trihydrate of the gibbsite structure, and the beta-alumina trihydrate of the bayerite structure. A suitable alumina is available from UOP LLC of Des Plaines, IL under the trade designation VERSAL. A preferred alumina is available from Sasol North America Alumina Product Group under the trade designation CATAPAL. Typically, this material is an extremely high purity alpha-alumina monohydrate (pseudo-boehmite) which after calcination at a high temperature has been shown to yield a high purity gamma-alumina.

A suitable oligomerization catalyst may be prepared by mixing proportionate volumes of zeolite and alumina to achieve the desired zeolite-to-alumina ratio. In an embodiment, 5 to 80, typically 10 to 60, suitably 15 to 40, and preferably 20 to 30%, by weight, MTT zeolite and the balance alumina powder provides a suitably supported catalyst. A silica support is also contemplated.

A monoprotic acid such as nitric acid or formic acid may be added to the mixture in aqueous solution to peptize the alumina in the binder. Additional water can be added to the mixture to provide sufficient wetness to constitute a dough with sufficient consistency to be extruded or spray dried. Optionally, extrusion aids such as cellulose ether powders can also be added. A preferred extrusion aid is available from The Dow Chemical Company of Midland, MI under the trade designation METHOCEL.

The paste or dough may be prepared in the form of shaped particulates, with the preferred method being to extrude the dough through a die having openings therein of desired size and shape, after which the extruded matter can be broken into extrudates of desired length and dried. A further step of calcination may be employed to give added strength. Generally, calcination is conducted in a stream of dry air at a temperature of 260° to 815°C. The MTT catalyst need not be selectively activated to neutralize acid sites such as with an amine.

The extruded particles may have any suitable cross-sectional shape, i.e., symmetrical or asymmetrical, but most often have a symmetrical cross-sectional shape, preferably a spherical, cylindrical or polylobal shape. The cross-sectional diameter of the particles may be as small as 40 µm; however, it is usually 0.635 to 12.7 mm, preferably 0.79 to 6.35 mm, and alternatively 0.06 to 4.23 mm.

Exemplary oligomerization reaction zones are disclosed in, e.g., US 5,895,830. The oligomerization reaction zone 600 can produce an oligomerization effluent 620. Preferably, the oligomerization effluent 620 may include one or more C8 alkenes, such as linear C8 alkenes for producing propene in the FCC apparatus 200 and branched C8 alkenes for blending as alkylate. Other compounds may be present. As such, the oligomerization effluent 620 includes one or more C8⁺ hydrocarbons, such as oligomerized C3-C6 hydrocarbons. These oligomerized C3-C6 hydrocarbons include at least one C8-C16 alkene, although other alkenes may also be present as well as alkanes. Preferably, the oligomerization reaction zone 600 produces one or more light alkene oligomeric hydrocarbons, more preferably one or more linear and branched C8 hydrocarbons, as well as a C4 stream 610, which can optionally be recycled to the oligomerization reaction zone 600.

The oligomerization effluent 620 can contain different mixtures of linear or branched hydrocarbons. In some embodiments, it is desirable to produce more propene in the fluid catalytic cracking apparatus 200. In such instances, it may be desirable to have more linear alkenes in the oligomerization effluent 620. In other instances, maximizing fuel octane rating is desired by inclusion of branched hydrocarbons. Exemplary C8 alkene fractions, in weight percent, for oligomerization effluents made with, respectively, SPA and MTT catalysts are depicted below:

**TABLE 1**

| C8 Compound | SPA Catalyst | MTT Catalyst |
|---|---|---|
| Linear Octene | 0 | 4 |
| Methylheptene | 3 | 30 |
| Dimethylhexene | 23 | 37 |
| Trimethyl pentene | 74 | 29 |

If producing more propene is desired, MTT catalyst can produce more linear octene or less branched C8 alkenes as compared to other catalysts. In such instances, providing such an oligomerization effluent to a riser with a residence time of no more than 0.3 seconds, such as 0.01 to 0.1 seconds or even 0.05 seconds, can result in greater amounts of propene. Generally, a weight ratio of 0.1:1 to 1.2:1 or 0.8:1 to 1.2:1 of linear alkene, e.g., octene, as compared to a branched alkene species, e.g., methylheptene, dimethylhexene, or trimethyl pentene, is desirable. Alternatively, the linear alkene species, e.g., octene, can comprise 3% to 45%, by weight, of the total alkenes for a numerated hydrocarbon. As an example, octene can comprise 3% to 45%, by weight, of the C8 alkenes. Further still, the MTT oligomerate may be less branched even if linear alkenes are not prevalent. In such an instance, the less branched MTT oligomerate may be more easily cracked.

If another product is desired, such as a fuel product with high octane, the oligomerization catalyst can be chosen to maximize branched alkenes. In this instance, a SPA catalyst may be selected. Thus, changing the oligomerization zone catalyst can provide flexibility and produce the desired ratios of linear and branched alkenes to maximize the desired products from the fluid catalytic cracking apparatus, such as branched hydrocarbons for improving octane or additional amounts of propene.

Generally, the oligomerization effluent 620 can be split into a recycle stream 382, which can serve as a second feed 382, and a product stream 640. The product stream 640 can be rich in C8⁺ alkylate and be sent to any suitable destination, such as a gasoline blending pool, to improve gasoline octane. The recycle stream 382 including one or more linear and branched C8⁺ hydrocarbons may be provided to the FCC apparatus 200, as the second feed 382.

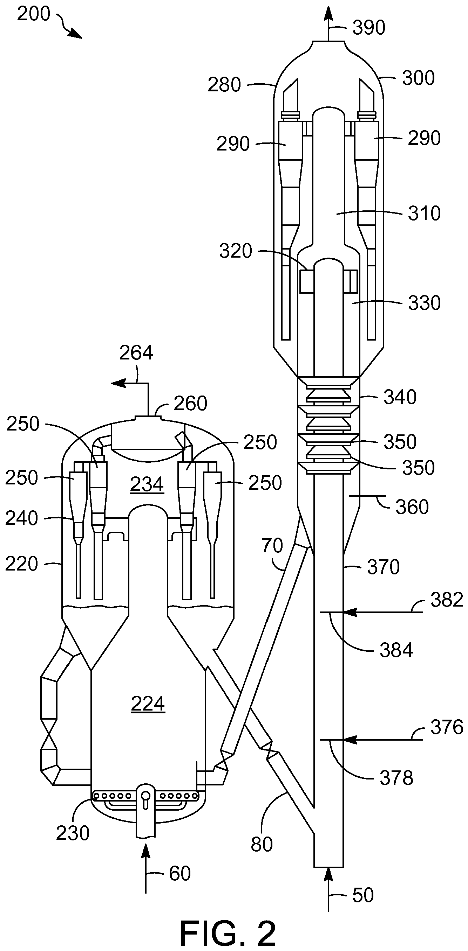

Referring to FIG. 2, the FCC apparatus 200 can include a regenerator 220 and a riser-reactor 280. Generally, the first feed 376 can be provided at a first, lower elevation through a first distributor 378 and the second feed 382 can be provided at a second, higher elevation through a second distributor 384. The distributors 378 and 384 can be any suitable device, such as a pipe having a series of holes a circumference.

Typically, the first feed 376 may be a heavy hydrocarbon having a boiling point range of 180° to 800°C. The first feed 376 can be at least one of a gas oil, a vacuum gas oil, an atmospheric gas oil, an atmospheric residue, and a vacuum residue. Alternatively, the first feed 376 may be at least one of a heavy cycle oil and a slurry oil. The feed may have a temperature of 140° to 430°C, preferably 200° to 290°C and a residence time of 2 to 5 seconds. The second feed 382 includes one or more compounds as discussed above for the oligomerization effluent 620 depicted in FIG. 1. The second feed 382 has a residence time of 0.01 to 0.3 second. Depending on the desired feed and products, it can be advantageous to have a short residence time, such as no more than 0.3 second, if the feed contains suitable amounts of linear alkenes, such as octene, or less branched alkene species.

The riser-reactor 280 can include the riser 370 terminating in a chamber 330 housed in a separation vessel 300. Although the separation vessel 300 can facilitate the separation between hydrocarbons and catalyst, reactions may continue to occur in the separation vessel 300. A lift gas stream 50, such as steam and/or a light hydrocarbon, may be provided to the bottom of the riser 370. A catalyst may be provided via a regenerator standpipe 80, with additional catalyst provided via a make-up line.

The catalyst can be a single catalyst or a mixture of different catalysts. A single catalyst may be used, such as a medium or smaller pore zeolite catalyst, such as an MFI zeolite, as exemplified by at least one of ZSM-5, ZSM-11, ZSM-12, ZSM-23, ZSM-35, ZSM-38, ZSM-48, and other similar materials, preferably the catalyst includes ZSM-5. Other suitable medium or smaller pore zeolites include ferrierite, and erionite. Preferably, the medium or smaller pore zeolite dispersed on a matrix includes a binder material such as silica or alumina and an inert filler material such as kaolin. The catalyst may also include some other active material such as Beta zeolite. These compositions may have a crystalline zeolite content of 10 to 50 wt% or more, and a matrix material content of 50 to 90 wt%. Generally, medium and smaller pore zeolites are characterized by having an effective pore opening diameter of less than or equal to 0.7 nm, rings of 10 or fewer members, and a Pore Size Index of less than 31. Alternatively, a catalyst mixture may be used as disclosed in, e.g., US 7,312,370 B2 and US 2010/0236980 A1. In one preferred embodiment, the catalyst mixture can include a Y zeolite and a ZSM-5 zeolite.

Typically, the riser 370 operates with dilute phase conditions above the point of feed injection with a density that is less than 320 kg/m³. Usually, the feeds 376 and 382 are injected above the catalyst provided by the regenerator standpipe 80 and the lift gas stream 50. Moreover, additional amounts of feed may also be introduced upstream or downstream of either of the feeds 376 and 382.

Also, the riser-reactor 280 in one desired embodiment can be operated at low hydrocarbon partial pressure. Generally, a low hydrocarbon partial pressure can facilitate the production of light alkenes, such as C2 and/or C3 alkene. Accordingly, the riser 370 pressure can be 170 to 250 kPa, with a hydrocarbon partial pressure of 35 to 180 kPa, preferably 70 to 140 kPa. A relatively low partial pressure for hydrocarbon may be achieved by using steam as a diluent, in the amount of 10 to 55 wt%, preferably 15 wt%, based on the feed. Other diluents, such as dry gas, can be used to reach equivalent hydrocarbon partial pressures.

The one or more hydrocarbons and catalyst rise to the chamber 330 converting the feeds 376 and 382. The riser 370 can operate at any suitable temperature, and typically operates at a riser outlet temperature of 400° to 600°C, preferably 500° to 600°C. Exemplary risers are disclosed in, e.g., US 5,154,818 and US 4,090,948.

The products can rise within the riser 370 and exit within the chamber 330. Typically, products including propene and gasoline are produced. Subsequently, the catalyst can separate assisted by any suitable device, such as swirl arms 320, and settle to the bottom of the separation vessel 300. In contrast, one or more products and any remaining entrained catalyst can rise within a gas conduit 310. The entrained catalyst can be separated using separation devices, such as one or more cyclone separators 290 for separating out the products from the catalyst particles. Dip legs may drop the catalyst down to the base of the separation vessel 300 where openings can permit entry of the spent catalyst into a dense catalyst bed. Exemplary separation devices and swirl arms are disclosed in, e.g., US 7,312,370 B2.

The one or more products leaving the cyclone separators 290 can exit as an effluent 390 from the riser-reactor 280, and be provided to the separation zone 400, as described above.

With respect to the catalyst separated by the cyclone separators 290, the catalyst can fall to a stripping zone 340. The catalyst may pass through the stripping zone 340 over baffles 350 where absorbed hydrocarbons can be removed from the surface of this catalyst by counter-current contact with steam provided via a line 360. An exemplary stripping zone is disclosed in, e.g., US 7,312,370 B2. Catalyst may pass via a catalyst conduit 70 to the regenerator 220.

The regenerator 220 can include two stages 224 and 234, and have a regenerator distributor 230 in the first stage 224; and a tee disengager 240, regenerator cyclones 250, and an outlet 260 in the second stage 234. Typically, an oxidizing stream 60, such as an air stream, can be provided to the distributor 230 for combusting the catalyst. The gases and catalyst can rise in the first stage 224 and exit the tee disengager 240 of the second stage 234. Most of the catalyst can fall and be passed via the regenerator standpipe 80 to the riser 370. The fine catalyst and gases can enter the regenerator cyclones 250 to further separate catalyst from flue gases. The catalyst can be directed to the bottom of the second stage 234 and the regenerator standpipe 80. The flue gases can pass to the outlet 260 and pass from the regenerator 220 as a flue gas stream 264. Exemplary regeneration vessels are disclosed in, e.g., US 7,312,370 B2 and 7,247,233 B1.

### ILLUSTRATIVE EMBODIMENTS

The following examples are intended to further illustrate the subject process. These illustrations of embodiments of the invention are not meant to limit the claims of this invention to the particular details of these examples. These examples are based on engineering calculations and actual operating experience with similar processes.

### EXAMPLE 1 (not according to the invention)

Two runs are conducted with a feed of 1-octene at different residence times. The first run is conducted in a microreactor with two samples at a shorter residence time. The second run is conducted in a pilot plant with a sample at a longer residence time. Each of these runs utilize a feed of 1-octene and are conducted with a catalyst weight ratio of 90/10 of Y/ZSM-5 zeolite. The conditions are as follows:

**TABLE 2**

| Parameter | Run 1 | | Run 2 |
|---|---|---|---|
| Temperature, °C | 565 | 565 | 593 |
| Pressure, kPa | 112 | 112 | 38 |
| Residence Time (seconds) | 0.048 | 0.048 | 0.74 |

**TABLE 3**

| Reaction Results | Run 1 | | Run 2 |
|---|---|---|---|
| Conversion 1-Octene, wt% | 96.8 | 96.8 | 98.1 |
| Yields, wt% | | | |
| C3⁻ | 2.4 | 2.5 | 7.3 |
| Propene | 26.3 | 26.5 | 22.6 |
| C4 (Alkane and Alkene) | 37.6 | 37.5 | 33.8 |
| C5-C7 | 27.2 | 27.0 | 25.1 |
| 1-Octene | 3.2 | 3.2 | 1.9 |
| Other C8⁺ | 3.3 | 3.3 | 9.3 |

The data demonstrates higher yield (over 26% for Run 1 versus less than 23% for Run 2) for propene at short residence times when cracking 1-octene.

Two additional runs are conducted with a third feed of oligomerized product made by reacting one or more C4 hydrocarbons over a catalyst including 20%, by weight, MFI zeolite and 80%, by weight, alumina, and a fourth feed being a mixture of the oligomerized product and a vacuum gas oil at different residence times. The oligomerized product has the following composition:

**TABLE 4**

| Species | Wt% |
|---|---|
| Butane | 6.57 |
| Butene | 7.46 |
| Pentane | 0.01 |
| Pentene | 0.68 |
| C7⁺ Alkenes | 85.28 |

The fourth feed includes 24%, by weight, of the oligomerized product and 76%, by weight, of the vacuum gas oil. The third and fourth runs are conducted with a catalyst weight ratio of 90/10 of Y/ZSM-5 zeolite, and in a pilot plant. The conditions are as follows:

**TABLE 5**

| Parameter | Run 3 | Run 4 |
|---|---|---|
| Temperature, °C | 593 | 565 |
| Pressure, kPa | 40.7 | 379 |
| Residence Time, seconds | 0.81 | 2.4 |

**TABLE 6**

| Reaction Results | Run 3 | Run 4 |
|---|---|---|
| Feed | Third | Fourth |
| Oligomerized Product Feed Conversion, wt% | 99.0 | 100.0 |
| Yields, wt% | | |
| C3⁻ | 6.5 | 7.5 |
| Propene | 16.7 | 11.0 |
| C4 (Alkane and Alkene) | 53.2 | 28.0 |
| C5-C7 | 17.5 | 40.0 |
| 1-Octene | 1.0 | 0.0 |
| Other C8⁺ | 5.1 | 13.5 |

The data demonstrates a higher yield for propene at shorter residence times (over 16% for Run 3 versus 11% for Run 4) when cracking oligomerized product.

Although not wanting to be bound by theory, a lower residence time can provide a high selectivity for propene with respect to linear alkenes and lesser branched alkenes. A higher residence time can result in less selectivity for propene. A higher residence time appears to result in undesirable hydrogen transfer reactions that can consume alkenes. A lower residence time over the FCC catalyst with ZSM-5 appears to prevent the cracking of highly branched octenes that crack primarily back to undesirable C4 alkenes. Moreover, a highly branched C8 alkene can have a high octane value and preservation is generally preferred over cracking back to butene. Creating additional butenes is usually undesired due to the increase of recycling of butenes in a unit for processing hydrocarbons.

Thus, the embodiments disclosed herein can preserve highly branched, high octane C8 alkenes, such as 2,4,4 trimethyl pentene over, e.g., a ZSM-5 zeolite or a combination of a ZSM-5/Y zeolite. Moreover, linear C8 alkenes, such as 1-octene, and lesser branched alkenes can be cracked rapidly at shorter residence times to produce propene.

### EXAMPLE 2

Several oligomerization effluents are obtained from a pilot plant and tested in a fixed bed micro reactor system to simulate fluid catalytic cracking. The first oligomerization reaction effluent is obtained using a SPA catalyst (Sample A) and the second oligomerization reaction effluent is obtained using a catalyst including an MTT zeolite (Samples B and C). The first oligomerization reaction effluent is a blended material boiling greater than 80° C to obtain a Sample A. The second oligomerization reaction effluent is divided into Samples B and C with Sample B being a cut boiling greater than 150° C and Sample C being an unfractionated blend. Sample A has the following composition:

**TABLE 7**

| | | | |
|---|---|---|---|
| Component | C6 | C7 | C8⁺ |
| Wt% | 1.39 | 0.72 | 97.88 |

Sample B has the following composition:

**TABLE 8**

| | | |
|---|---|---|
| Component | C8 | C9⁺ |
| Wt% | 0.1 | 99.9 |

Sample C has the following composition:

**TABLE 9**

| | | | | | | |
|---|---|---|---|---|---|---|
| Component | Butene | C5 | C6 | C7 | C8 | Benzene |
| Wt% | 0.07 | 0.04 | 0.23 | 1.24 | 98.41 | 0.01 |

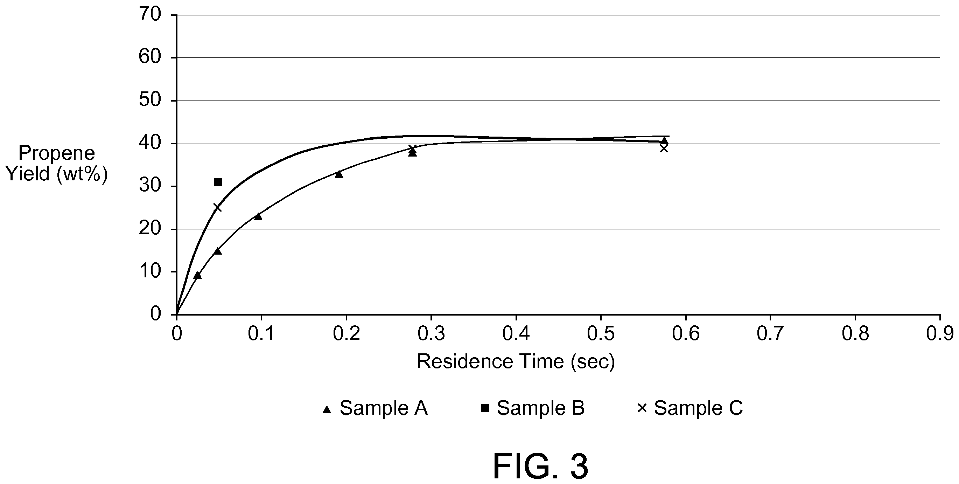

Each oligomerization reaction effluent is fed to a pilot plant under substantially similar conditions, namely a temperature of 565°C and a residence time of 0.024 to 0.6 second. Each hydrocarbon feed is at a concentration 10%, by volume, hydrocarbon, 3%, by volume, steam, and the balance nitrogen. Each sample is contacted with a catalyst mixture of 25%, by weight, ZSM-5 zeolite catalyst and 75%, by weight, Y-zeolite catalyst. Referring to FIG. 3, Samples B and C provide greater propene yield at a residence time less than 0.3 second, or less than 0.1 second, as compared to Sample A. Hence, the oligomerization effluent from the MTT catalyst produces greater propene yield at lower residence times.

### EXAMPLE 3

Five samples of oligomerization effluent are tested in a fluid catalytic cracking pilot plant under similar conditions. A Y-zeolite based equilibrium catalyst contains 12%, by weight, ZSM-5 zeolite. The feed to the fluid catalytic cracking pilot plant includes a vacuum gas oil with an oligomer recycle. The feed can also include 25%, by weight, of an oligomerization reaction effluent. The oligomerization reaction effluent can be produced from a SPA catalyst or a catalyst including an MTT zeolite. If the oligomerization reaction effluent is included, the oligomerization reaction effluent can be provided separately high or combined low with the vacuum gas oil on the riser. If provided high on the riser, then the oligomerization reaction effluent is reacted at a shorter residence time as compared to low on the riser. Thus, Sample D is a vacuum gas oil provided low on the riser. Sample E is a combined feed of a vacuum gas oil and 25%, by weight, of an oligomerization reaction effluent obtained from a SPA catalyst and provided low on the riser. Sample F is a feed of a vacuum gas oil provided low on the riser and 25%, by weight, of an oligomerization reaction effluent obtained from a MTT catalyst provided separately high on the riser. Sample G is a feed of a vacuum gas oil provided low on the riser and 25%, by weight, of an oligomerization reaction effluent obtained from a SPA catalyst provided separately high on the riser. Sample H is a combined feed of a vacuum gas oil and 25%, by weight, of an oligomerization reaction effluent obtained from a MTT catalyst provided low on the riser.

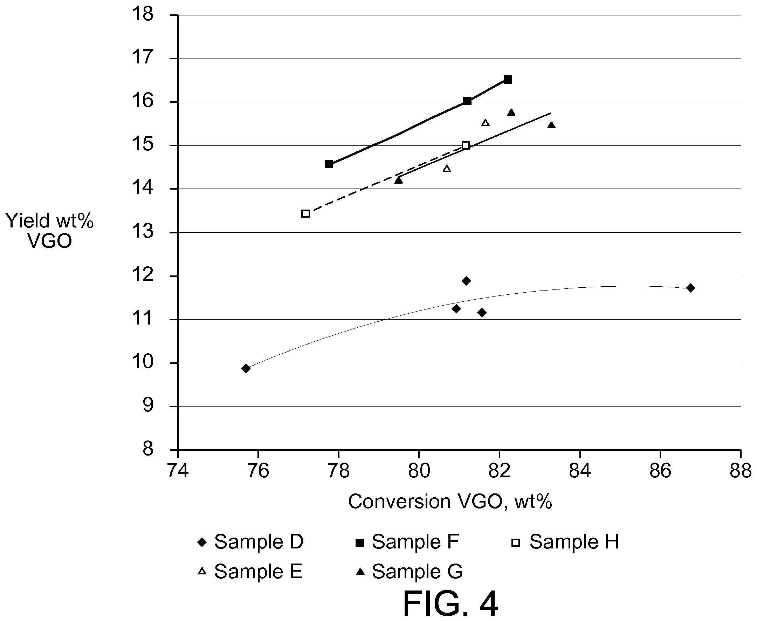

Referring to FIG. 4, the five samples are compared. Sample D is a vacuum gas oil that can provide a propene yield based on weight percent of vacuum gas oil of 10-12%, versus vacuum gas oil conversion. Sample F, which is an oligomerization reaction effluent made from a MTT zeolite catalyst, results in a propene yield of 14-17%, by weight, vacuum gas oil. This propene yield is higher than Samples E and G having 25%, by weight, oligomerization reaction effluent obtained by using a SPA catalyst. Thus, using the oligomerization reaction effluent from a MTT zeolite catalyst and providing such an effluent high on a riser with a shorter residence time can boost propene production as compared to other oligomerization reaction effluents and/or locations.

Without further elaboration, it is believed that one skilled in the art can, using the preceding description, utilize the present invention to its fullest extent. The preceding preferred specific embodiments are, therefore, to be construed as merely illustrative, and not limitative of the remainder of the disclosure in any way whatsoever.

In the foregoing, all temperatures are set forth in degrees Celsius and, all parts and percentages are by weight, unless otherwise indicated.

### SPECIFIC EMBODIMENTS

While the following is described in conjunction with specific embodiments, it will be understood that this description is intended to illustrate and not limit the scope of the preceding description and the appended claims.

A first embodiment of the invention is a process for fluid catalytic cracking, comprising A) providing a first feed comprising one or more heavy hydrocarbons to a riser of a riser-reactor; B) obtaining a second feed from an oligomerization zone wherein the second feed comprises one or more light alkene oligomeric hydrocarbons; and C) providing the second feed downstream from the first feed for producing propene. An embodiment of the invention is one, any or all of prior embodiments in this paragraph up through the first embodiment in this paragraph, wherein the one or more heavy hydrocarbons comprises at least one of a gas oil, a vacuum gas oil, an atmospheric gas oil, an atmospheric residue, a vacuum residue, a heavy cycle oil, and a slurry oil. An embodiment of the invention is one, any or all of prior embodiments in this paragraph up through the first embodiment in this paragraph, further comprising oligomerizing one or more light alkenes comprising at least one C4 and C5 alkene for producing the one or more light alkene oligomeric hydrocarbons. An embodiment of the invention is one, any or all of prior embodiments in this paragraph up through the first embodiment in this paragraph, further comprising passing an effluent from the riser-reactor to a separation zone. An embodiment of the invention is one, any or all of prior embodiments in this paragraph up through the first embodiment in this paragraph, wherein the separation zone provides a stream comprising one or more light alkenes. An embodiment of the invention is one, any or all of prior embodiments in this paragraph up through the first embodiment in this paragraph, further comprising providing the one or more light alkenes to an oligomerization zone producing one or more light alkene oligomeric hydrocarbons. An embodiment of the invention is one, any or all of prior embodiments in this paragraph up through the first embodiment in this paragraph, wherein the oligomerization zone is at a temperature of 30° to 300°C and at a pressure of 790 to 8,400 kPa. An embodiment of the invention is one, any or all of prior embodiments in this paragraph up through the first embodiment in this paragraph, wherein the oligomerization zone is at a temperature of 160° to 220°C and at a pressure of 3,400 to 6,400 kPa. An embodiment of the invention is one, any or all of prior embodiments in this paragraph up through the first embodiment in this paragraph, wherein the one or more light alkene oligomeric hydrocarbons comprises at least one C8-C16 alkene. An embodiment of the invention is one, any or all of prior embodiments in this paragraph up through the first embodiment in this paragraph, wherein a residence time for the first feed is 2 to 5 seconds. An embodiment of the invention is one, any or all of prior embodiments in this paragraph up through the first embodiment in this paragraph, wherein the first feed and the second feed are reacted in a presence of a catalyst comprising Y zeolite or a combination of Y and ZSM-5 zeolites.

A second embodiment of the invention is a process for fluid catalytic cracking, comprising A) providing a first feed comprising one or more heavy hydrocarbons to a riser of a riser-reactor; B) obtaining a second feed from an oligomerization zone wherein the second feed comprises one or more light alkene oligomeric hydrocarbons; C) providing the second feed at a higher elevation to the riser and downstream from the first feed for producing a product comprised in an effluent; and D) providing the effluent to a separation zone. An embodiment of the invention is one, any or all of prior embodiments in this paragraph up through the second embodiment in this paragraph, wherein a residence time for the first feed is 2 to 5 seconds. An embodiment of the invention is one, any or all of prior embodiments in this paragraph up through the second embodiment in this paragraph, wherein a residence time for the second feed is no more than 0.3 second. An embodiment of the invention is one, any or all of prior embodiments in this paragraph up through the second embodiment in this paragraph, wherein the one or more heavy hydrocarbons comprises at least one of a gas oil, a vacuum gas oil, an atmospheric gas oil, an atmospheric residue, and a vacuum residue.

A third embodiment of the invention is a process for fluid catalytic cracking, comprising A) providing a first feed comprising one or more heavy hydrocarbons to a riser operated at a riser outlet temperature of 400° to 600°C of a riser-reactor; B) providing a second feed comprising one or more light alkene oligomeric hydrocarbons at a higher elevation to the riser from the first feed for producing propene in an effluent; C) providing the effluent to a separation zone to obtain one or more light alkenes; and D) providing the one or more light alkenes comprising butene to an oligomerization reaction zone operating at a temperature of 30° to 260°C to obtain one or more light alkene oligomeric hydrocarbons wherein at least some of the one or more light alkene oligomeric hydrocarbons is provided as the second feed. An embodiment of the invention is one, any or all of prior embodiments in this paragraph up through the third embodiment in this paragraph, wherein the oligomerization reaction zone contains a catalyst comprising an MTT zeolite.

Without further elaboration, it is believed that one skilled in the art can, using the preceding description, utilize the present invention to its fullest extent. The preceding preferred specific embodiments are, therefore, to be construed as merely illustrative, and not limitative of the remainder of the disclosure in any way whatsoever.

In the foregoing, all temperatures are set forth in degrees Celsius and, all parts and percentages are by weight, unless otherwise indicated.

## Claims

1. A process for fluid catalytic cracking, comprising:
A) providing a first feed comprising one or more heavy hydrocarbons to a riser of a riser-reactor; wherein the one or more heavy hydrocarbons comprises at least one of a gas oil, a vacuum gas oil, an atmospheric gas oil, an atmospheric residue, a vacuum residue, a heavy cycle oil, and a slurry oil;
B) oligomerizing, in an oligomerization zone in the presence of an oligomerization catalyst comprising an MTT zeolite, one or more light alkenes comprising at least one C₄ or C₅ alkene for producing one or more light alkene oligomeric hydrocarbons;
C) obtaining a second feed from the oligomerization zone wherein the second feed comprises the one or more light alkene oligomeric hydrocarbons; wherein the one or more light alkene oligomeric hydrocarbons comprises at least one C₈-C₁₆ alkene; and
D) providing the second feed downstream from the first feed and cracking said feed to produce propene; wherein the second feed is provided to the riser with a residence time of from 0.01 to no more than 0.3 seconds.

2. The process according to claim 1, further comprising passing an effluent from the riser-reactor to a separation zone.

3. The process according to claim 2, wherein the separation zone provides a stream comprising one or more light alkenes.

4. The process according to claim 3, further comprising providing the one or more light alkenes to an oligomerization zone producing one or more light alkene oligomeric hydrocarbons.

5. The process according to claim 4 wherein the oligomerization zone is at a temperature of 30° to 300°C and at a pressure of 790 to 8,400 kPa.

6. The process according to claim 4 wherein the oligomerization zone is at a temperature of 160° to 220°C and at a pressure of 3,400 to 6,400 kPa.

7. The process according to claim 1, wherein a residence time for the first feed is 2 to 5 seconds.

## Patentansprüche

1. Verfahren zum katalytischen Cracken in der Wirbelschicht, das Folgendes umfasst:
A) Zuführen eines ersten Einsatzstoffs, der einen oder mehrere schwere Kohlenwasserstoffe umfasst, zu einem Steigrohr eines Steigrohrreaktors; wobei der eine bzw. die mehreren schweren Kohlenwasserstoffe ein Gasöl, ein Vakuumgasöl, ein atmosphärisches Gasöl, einen atmosphärischen Rückstand, einen Vakuumrückstand, ein Heavy Cycle Oil und/oder ein Slurry Oil umfasst bzw. umfassen;
B) Oligomerisieren eines oder mehrerer leichter Alkene, die mindestens ein C₄- oder C₅-Alken umfassen, in einer Oligomerisationszone in Gegenwart eines Oligomerisationskatalysators, der einen MTT-Zeolith umfasst, zur Herstellung eines oder mehrerer oligomerer Kohlenwasserstoffe leichter Alkene;
C) Erhalten eines zweiten Einsatzstoffs aus der Oligomerisationszone, wobei der zweite Einsatzstoff den einen bzw. die mehreren oligomeren Kohlenwasserstoffe leichter Alkene umfasst; wobei der eine bzw. die mehreren oligomeren Kohlenwasserstoffe leichter Alkene mindestens ein C₈-C₁₆-Alken umfasst bzw. umfassen; und
D) Bereitstellen des zweiten Einsatzstoffs stromabwärts des ersten Einsatzstoffs und Cracken des Einsatzstoffs zur Herstellung von Propen; wobei der zweite Einsatzstoff dem Steigrohr mit einer Verweilzeit von 0,01 bis nicht mehr als 0,3 Sekunden zugeführt wird.

2. Verfahren nach Anspruch 1, ferner umfassend das Leiten eines Austragsstroms aus dem Steigrohrreaktor zu einer Trennzone.

3. Verfahren nach Anspruch 2, bei dem die Trennzone einen Strom, der ein oder mehrere leichte Alkene umfasst, bereitstellt.

4. Verfahren nach Anspruch 3, ferner umfassend das Zuführen des einen bzw. der mehreren leichten Alkene zu einer Oligomerisationszone unter Herstellung eines oder mehrerer oligomerer Kohlenwasserstoffe leichter Alkene.

5. Verfahren nach Anspruch 4, bei dem sich die Oligomerisationszone bei der Temperatur von 30 bis 300 °C und einem Druck von 790 bis 8400 kPa befindet.

6. Verfahren nach Anspruch 4, bei dem sich die Oligomerisationszone bei der Temperatur von 160 bis 220 °C und einem Druck von 3400 bis 6400 kPa befindet.

7. Verfahren nach Anspruch 1, bei dem die Verweilzeit für den ersten Einsatzstoff 2 bis 5 Sekunden beträgt.

## Revendications

1. Procédé de craquage catalytique en lit fluidisé, comprenant :
A) l'introduction d'une première charge d'alimentation comprenant un ou plusieurs hydrocarbures lourds dans une colonne montante d'un réacteur à colonne montante ; le ou les hydrocarbures lourds comprenant au moins l'un d'un gasoil, d'un gasoil sous vide, d'un gasoil atmosphérique, d'un résidu de distillation atmosphérique, d'un résidu de distillation sous vide, d'une huile de craquage catalytique lourde et d'un résidu de craquage catalytique ;
B) l'oligomérisation, dans une zone d'oligomérisation en présence d'un catalyseur d'oligomérisation comprenant une zéolite MTT, d'un ou plusieurs alcènes légers comprenant au moins un alcène en C₄ ou en C₅ pour la production d'un ou plusieurs hydrocarbures oligomères alcéniques légers ;
C) l'obtention d'une seconde charge d'alimentation à partir de la zone d'oligomérisation, la seconde charge d'alimentation comprenant le ou les hydrocarbures oligomères alcéniques légers ; le ou les hydrocarbures oligomères alcéniques légers comprenant au moins un alcène en C₈-C₁₆ ; et
D) l'introduction de la seconde charge d'alimentation en aval de la première charge d'alimentation et le craquage de ladite charge d'alimentation pour produire du propène ; la seconde charge d'alimentation étant introduite dans la colonne montante avec un temps de séjour de 0,01 à pas plus de 0,3 seconde.

2. Procédé selon la revendication 1, comprenant en outre le passage d'un effluent du réacteur à colonne montante vers une zone de séparation.

3. Procédé selon la revendication 2, dans lequel la zone de séparation fournit un flux comprenant un ou plusieurs alcènes légers.

4. Procédé selon la revendication 3, comprenant en outre l'introduction du ou des alcènes légers dans une zone d'oligomérisation produisant un ou plusieurs hydrocarbures oligomères alcéniques légers.

5. Procédé selon la revendication 4, dans lequel la zone d'oligomérisation est à une température de 30° à 300 °C et à une pression de 790 à 8 400 kPa.

6. Procédé selon la revendication 4, dans lequel la zone d'oligomérisation est à une température de 160° à 220 °C et à une pression de 3 400 à 6 400 kPa.

7. Procédé selon la revendication 1, dans lequel un temps de séjour pour la première charge d'alimentation est de 2 à 5 secondes.
